# EUROPEAN PATENT APPLICATION

(11) **EP 4 310 185 A1**
(43) Date of publication of application: **24.01.2024**
(21) Application number: 22766702.9
(22) Date of filing: 08.02.2022
(51) Int. Cl.: C12N 15/12, G01N 33/53, G01N 33/543

(54) **METHOD AND DEVICE FOR MEASURING TDP-43 IN BIOLOGICAL SAMPLE**

(30) Priority: 10.03.2021 JP 2021038665
(71) Applicant: National Institutes for Quantum Science and Technology, Chiba 263-8555 (JP)
(72) Inventor: TOKUDA, Takahiko, Chiba-shi, Chiba 263-8555 (JP); TATEBE, Harutsugu, Chiba-shi, Chiba 263-8555 (JP)
(74) Representative: KATZAROV S.A.
(86) International application number: PCT/JP2022/004776
(87) International publication number: WO 2022/190737

(57) **Abstract**

The present invention relates to a method for measuring TDP-43 in a biological sample. Non-capture beads used in this method each do not bind to an immune complex of TDP-43 in a biological sample, a capture antibody, and a detection antibody. The immune complex is formed on each of capture beads. At least one of the detection antibody and the capture antibody specifically recognizes the TDP-43. An epitope of the capture antibody and an epitope of the detection antibody differ from each other.

## Description

### Technical Field

The present invention relates to a method, a device, etc. for measuring TDP-43 in a biological sample collected from a subject.

### Background Art

TDP-43 is known as an RNA-binding protein. TDP-43 is also considered to be one of candidate biomarkers for diseases such as frontotemporal lobar degeneration (FTLD) and amyotrophic lateral sclerosis (ALS).

For example, Non-Patent Literature 1 discloses that ELISA was used to measure concentrations of TDP-43 in spinal fluid and the concentrations of TDP-43 significantly increased in ALS patients. Non-Patent Literature 3 discloses that ELISA was used to measure concentrations of TDP-43 in blood plasma of FTLD patients.

Non-patent reference 2 discloses that Simoa (trademark), which is known as an ultrasensitive digital ELISA device, was used to measure concentrations of neurofilament light chains (NfL) and TDP-43 in spinal fluid or blood plasma of ALS patients. Non-patent reference 2 also discloses that, in this measurement, a TDP-43 measurement kit (TDP43 Discovery Kit) dedicated to Simoa (trademark) was used in accordance with a protocol of the kit.

### Citation List

### [Non-patent Literature]

[Non-patent Literature 1]
   Kasai T, et al., Acta Neuropathol. 2009; 117(1): 55-62
[Non-patent Literature 2]
   Kasai T, et al., Ann Clin Transl Neurol. 2019; 6(12): 2489-2502
[Non-patent Literature 3]
   Foulds P, et al., Acta Neuropathol. 2008; 116(2): 141-6

### Summary of Invention

### Technical Problem

TDP-43 is present only in a very small amount in spinal fluid or blood plasma of a human. Therefore, the methods for measuring TDP-43 disclosed in the above described prior art documents each have low detection sensitivity. Therefore, the methods only allow detection in a small proportion of patients who have relatively high concentrations of TDP-43. In other words, at present, any significant difference is seen in an abundance of TDP-43 between a patient and a healthy subject, but the methods cannot be each used for an objective diagnosis of whether or not a subject has TDP-43 proteinopathy such as FTLD and ALS. Namely, in order to provide a way for TDP-43 to be used as a biomarker for a diagnosis, further improvement and development of a method and a device for measuring TDP-43 are desired.

The object of an aspect of the present invention is to realize a method, a device, etc. for measuring TDP-43, each of which has high detection sensitivity.

### Solution to Problem

In order to attain the above object, the present invention include the following aspects:
· A method for measuring TDP-43 in a biological sample, the method including:
   a preparing step of preparing a measurement sample including (i) non-capture beads and (ii) capture beads to each of which an immune complex of TDP-43 in a biological sample, a capture antibody, and a detection antibody binds, by forming the immune complex on each of the capture beads in the presence of the non-capture beads; and
   a detecting step of detecting a signal derived from the immune complex in the measurement sample, wherein
   each of the non-capture beads does not bind to the immune complex,
   at least one of the detection antibody and the capture antibody specifically recognizes the TDP-43,
   an epitope of the capture antibody and an epitope of the detection antibody differ from each other,
   a ratio of the number of the non-capture beads to the number of the capture beads is such that the non-capture beads are not less than 2.3 and not more than 9 in a case where the capture beads are regarded as 1, and
   a concentration of the capture beads and the non-capture beads is not less than 400,000 beads and not more than 500,000 beads with respect to 90 pL of the measurement sample.
· A method for measuring TDP-43 in a biological sample, the method including:
   a preparing step of preparing a measurement sample including (i) non-capture beads and (ii) capture beads to each of which an immune complex of TDP-43 in a biological sample, a capture antibody, and a detection antibody binds, by forming the immune complex on each of the capture beads in the presence of the non-capture beads; and
   a detecting step of detecting a signal derived from the immune complex in the measurement sample, wherein
   each of the non-capture beads does not bind to the immune complex,
   at least one of the detection antibody and the capture antibody specifically recognizes the TDP-43,
   an epitope of the capture antibody and an epitope of the detection antibody differ from each other,
   a ratio of the number of the non-capture beads to the number of the capture beads is such that the non-capture beads are not less than 2.3 and not more than 9 in a case where the capture beads are regarded as 1,
   a concentration of the capture beads and the non-capture beads is not less than 400,000 beads and not more than 500,000 beads with respect to 90 pL of the measurement sample, and
   EDTA is added to a buffer solution used for the capture beads and the non-capture beads.
· A device which measures TDP-43 in a biological sample collected from a subject, the device including
   a processing section, wherein
   the processing section obtains a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads,
   each of the non-capture beads does not bind to the immune complex,
   at least one of the detection antibody and the capture antibody specifically recognizes the TDP-43,
   an epitope of the capture antibody and an epitope of the detection antibody differ from each other,
   a ratio of the number of the non-capture beads to the number of the capture beads is such that the non-capture beads are not less than 2.3 and not more than 9 in a case where the capture beads are regarded as 1, and
   a concentration of the capture beads and the non-capture beads is not less than 400,000 beads and not more than 500,000 beads with respect to 90 pL of the measurement sample.
· A device which obtains information on whether or not a subject has TDP-43 proteinopathy, on the basis of an intensity of a signal derived from TDP-43 in a biological sample collected from the subject, the device including
   a processing section, wherein
   the processing section obtains a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads,
   each of the non-capture beads does not bind to the immune complex,
   at least one of the detection antibody and the capture antibody specifically recognizes the TDP-43,
   an epitope of the capture antibody and an epitope of the detection antibody differ from each other,
   the processing device obtains information on whether or not a subject has TDP-43 proteinopathy, on the basis of an intensity of the signal,
   a ratio of the number of the non-capture beads to the number of the capture beads is such that the non-capture beads are not less than 2.3 and not more than 9 in a case where the capture beads are regarded as 1, and
   a concentration of the capture beads and the non-capture beads is not less than 400,000 beads and not more than 500,000 beads with respect to 90 pL of the measurement sample.
· A computer program for measuring TDP-43, the computer program causing a computer to carry out
   a step of obtaining a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads, wherein
   the biological sample is collected from a subject,
   each of the non-capture beads does not bind to the immune complex,
   at least one of the detection antibody and the capture antibody specifically recognizes the TDP-43,
   an epitope of the capture antibody and an epitope of the detection antibody differ from each other,
   a ratio of the number of the non-capture beads to the number of the capture beads is such that the non-capture beads are not less than 2.3 and not more than 9 in a case where the capture beads are regarded as 1, and
   a concentration of the capture beads and the non-capture beads is not less than 400,000 beads and not more than 500,000 beads with respect to 90 pL of the measurement sample.
· A computer program for obtaining information on whether or not a subject has TDP-43 proteinopathy, the computer program causing a computer to carry out:
   a step of obtaining a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads; and
   a step of obtaining information on whether or not a subject from whom the biological sample has been collected has TDP-43 proteinopathy, on the basis of an intensity of the signal, wherein
   each of the non-capture beads does not bind to the immune complex,
   at least one of the detection antibody and the capture antibody specifically recognizes the TDP-43,
   an epitope of the capture antibody and an epitope of the detection antibody differ from each other,
   a ratio of the number of the non-capture beads to the number of the capture beads is such that the non-capture beads are not less than 2.3 and not more than 9 in a case where the capture beads are regarded as 1, and
   a concentration of the capture beads and the non-capture beads is not less than 400,000 beads and not more than 500,000 beads with respect to 90 pL of the measurement sample.

### Advantageous Effects of Invention

According to an aspect of the present invention, it is possible to realize a method, a device, etc. for measuring TDP-43, each of which has high detection sensitivity.

### Brief Description of Drawings

Fig. 1 is a graph showing a result of measuring a lower limit of a detectable concentration of a method for measuring TDP-43 in accordance with an embodiment of the present invention.
Fig. 2 is a graph showing a result of measuring a lower limit of a detectable concentration of a method for measuring TDP-43 by a conventional technique.
Fig. 3 is a graph showing results of measuring concentrations of TDP-43 in human spinal fluid samples by the method for measuring TDP-43 in accordance with an embodiment of the present invention.
Fig. 4 is a graph showing results of measuring concentrations of TDP-43 in human spinal fluid samples by the method for measuring TDP-43 by the conventional technique.
Fig. 5 is a graph showing a result of studying a concentration of non-capture beads.
Fig. 6 is a graph showing a result of studying the concentration of the non-capture beads.
Fig. 7 is a graph showing results of measuring concentrations of phosphorylated TDP-43 in human spinal fluid samples by the method for measuring TDP-43 in accordance with an embodiment of the present invention.
Fig. 8 is a view illustrating a configuration of a main part of a device which measures TDP-43 in accordance with an embodiment of the present invention.
Fig. 9 is a view illustrating a configuration of a main part of a device which obtains information on TDP-43 in accordance with an embodiment of the present invention.
Fig. 10 is a graph showing results of Evaluation Example 5.
Fig. 11 is a graph showing results of measuring concentrations of TDP-43 in blood samples of ALS patients and target patients in Evaluation Example 6.
Fig. 12 is a graph showing results of measuring concentrations of TDP-43 in blood samples of the ALS patients and AD patients in Evaluation Example 6.

### Description of Embodiments

### [1. Method for measuring TDP-43]

A method for measuring TDP-43 in accordance with an embodiment of the present invention (hereinafter, may be abbreviated to "measurement method in accordance with an embodiment of the present invention") is a method for measuring TDP-43 in a biological sample collected from a subject.

TDP-43 refers to TAR DNA-binding protein 43, and is an RNA/DNA-binding protein having two RNA recognition motifs. SEQ ID NO. 1 is an amino acid sequence of human-derived TDP-43. TDP-43 is involved in RNA metabolism including control of transcription, translation, and splicing. TDP-43 is mainly localized in a nucleus in a normal neuron. On the contrary, in a neuron of a patient with TDP-43 proteinopathy such as FTLD and ALS, phosphorylated TDP-43 is localized in a cytoplasm or a neurite.

Examples of human-derived TDP-43 include a protein which has an amino acid sequence registered as NCBI Reference Sequence NP_031401.

By the measurement method in accordance with an embodiment of the present invention, it is possible to measure at least one of non-phosphorylated TDP-43 and phosphorylated TDP-43 in the biological sample. In this specification, TDP-43 indicates both non-phosphorylated TDP-43 and phosphorylated TDP-43, unless otherwise specified. Phosphorylated TDP-43 is not limited, provided that TDP-43 is phosphorylated. Examples of phosphorylated TDP-43 include, but are not limited to, TDP-43 in which at least one of the 403th serine (Ser403), the 404th serine (Ser404), the 409th serine (Ser409), and the 410th serine (Ser410) in the amino acid sequence registered as NCBI Reference Sequence NP_031401 is phosphorylated.

Examples of the subject include mammals, birds, reptiles, and amphibians. Among others, the subject is preferably a mammal. Examples of the mammals include humans and non-human animals. Examples of the non-human animals include: domestic animals such as cattle, horses, pigs, and sheep; and pet animals and laboratory animals such as dogs, cats, rats, mice, hamsters, monkeys, and rabbits. Preferable is a human. Examples of the birds include poultry such as chickens, wild ducks, and domestic ducks.

The biological sample collected from the subject is not particularly limited, provided that the biological sample is a sample which can contain TDP-43. Examples of the biological sample include blood samples and spinal fluid (cerebrospinal fluid). The blood samples include whole blood, blood plasma, and blood serum. The blood samples are preferably blood plasma or blood serum, and more preferably blood plasma. Blood plasma can be separated from whole blood collected with a heparin salt (preferably sodium salt), a citrate (preferably sodium salt), or an ethylenediamine tetraacetic acid (EDTA) salt (preferably sodium salt or potassium salt). Blood plasma is more preferably blood plasma separated from whole blood collected with an EDTA salt or a heparin salt.

The measurement method in accordance with an embodiment of the present invention includes a preparing step and a detecting step. Each step is described below.

### (Preparing step)

In the preparing step, a measurement sample including (i) non-capture beads and (ii) capture beads to each of which an immune complex of TDP-43 in the biological sample, a capture antibody, and a detection antibody binds is prepared by forming the immune complex on each of the capture beads in the presence of the non-capture beads.

### <Antibodies>

Each antibody is not particularly limited, provided that the antibody can recognize TDP-43 (non-phosphorylated TDP-43 and/or phosphorylated TDP-43). The antibody which can recognize TDP-43 is preferably an antibody which specifically binds to TDP-43. The antibody which recognizes phosphorylated TDP-43 is more preferably an antibody which does not bind to non-phosphorylated TDP-43 but specifically binds to phosphorylated TDP-43.

As the antibody, any of a polyclonal antibody, a monoclonal antibody, and fragments thereof (e.g., Fab, F(ab)2, and the like) can be used. The polyclonal antibody and the monoclonal antibody are each obtained by immunizing a non-human animal with the whole or a part of TDP-43 as an antigen. Further, a class and a subclass of an immunoglobulin is not particularly limited. The antibody may be selected from an antibody library by a phage display method or the like.

The antibody may be a commercially available antibody. Examples of the antibody which recognizes non-phosphorylated TDP-43 include 60019-2-Ig (Proteintech Group, Inc.) and 12892-1-AP (Proteintech Group, Inc.). Examples of the antibody which recognizes phosphorylated TDP-43 include an antibody of which an antigen peptide is a peptide of phosphorylated TDP-43 which peptide corresponds to the 404th to 410th amino acids in the amino acid sequence of TDP-43 that is represented by SEQ ID NO. 1 (amino acid sequence registered as NCBI Reference Sequence NP_031401) and in which peptide the 409th and 410th serines (S) are phosphorylated.

An epitope of 60019-2-Ig above is a peptide composed of the 203rd to 209th amino acids in the amino acid sequence represented by SEQ ID NO. 1. An epitope of 12892-1-AP above is an epitope included in a peptide composed of the 261st to 414th amino acids in the amino acid sequence represented by SEQ ID NO. 1.

In the measurement method in accordance with an embodiment of the present invention, one of an epitope of the capture antibody and an epitope of the detection antibody preferably includes the peptide composed of the 203rd to 209th amino acids in the amino acid sequence represented by SEQ ID NO. 1. The other preferably includes (i) the peptide composed of the 261st to 414th amino acids in the amino acid sequence represented by SEQ ID NO. 1 or (ii) the peptide which is composed of the 404th to 410th amino acids in the amino acid sequence represented by SEQ ID NO. 1 and in which the 409th and 410th serines are phosphorylated. In measurement of non-phosphorylated TDP-43, one of the epitope of the capture antibody and the epitope of the detection antibody more preferably includes the peptide composed of the 203rd to 209th amino acids in the amino acid sequence represented by SEQ ID NO. 1. The other more preferably includes a peptide composed of the 261st to 414th amino acids in the amino acid sequence represented by SEQ ID NO. 1. In measurement of phosphorylated TDP-43, one of the epitope of the capture antibody and the epitope of the detection antibody more preferably includes the peptide composed of the 203rd to 209th amino acids in the amino acid sequence represented by SEQ ID NO. 1. The other preferably includes the peptide which is composed of the 404th to 410th amino acids and in which the 409th and 410th serines are phosphorylated.

In this specification, the capture antibody is an antibody which binds to TDP-43 and is immobilized on a bead described later. Immobilization on the bead may be indirect immobilization mediated by a substance such as avidin (or streptavidin) or biotin. In this specification, the detection antibody is an antibody which binds to TDP-43 and preferably has a labeling substance described later.

At least one of the detection antibody and the capture antibody is an antibody which specifically recognizes TDP-43. This makes it possible to specifically detect TDP-43 in the biological sample. The detection antibody and the capture antibody are each preferably an antibody which specifically recognizes TDP-43.

In a case where TDP-43 is phosphorylated TDP-43, at least one of the detection antibody and the capture antibody is an antibody that specifically recognizes phosphorylated TDP-43. The other may be an antibody which specifically recognizes phosphorylated TDP-43 or an antibody which specifically recognizes non-phosphorylated TDP-43.

The epitope of the detection antibody and the epitope of the capture antibody differ from each other.

### <Beads>

The beads are not limited, provided that the beads have a property that the beads can be spatially separated from each other. The beads may be provided in a form which allows the beads to be spatially separated in a plurality of locations (e.g., wells). For example, the beads may have a shape of a sphere, a disk, a ring, a cube, or the like. The beads may be fine particles (e.g., a plurality of particles suspended in a liquid), nanotubes, or the like.

The size or the shape of the beads may be designed as appropriate. For example, the shape of the beads includes a sphere, a cube, an ellipsoid, and a tube. The average diameter (in the case of a substantially spherical shape) or the average maximum cross-sectional size (in the case of another shape) of the beads may be not less than approximately 0.1 pm, not less than approximately 1 pm, not less than approximately 10 pm, not less than approximately 100 pm, or not less than approximately 1 mm. The average diameter or the maximum external size of the beads may be approximately 0.1 pm to approximately 100 pm, may be approximately 1 pm to approximately 100 pm, may be approximately 10 pm to approximately 100 pm, may be approximately 0.1 pm to approximately 1 mm, may be approximately 1 pm to approximately 10 mm, or may be approximately 0.1 pm to approximately 10 pm. The average diameter or the average maximum cross-sectional size of the beads used in the present embodiment is an arithmetic average value of diameters/maximum cross-sectional sizes of the beads.

The average maximum cross-sectional size of the beads can be measured with use of, for example, laser light scattering, microscopy, a sieve analysis, an electric resistance method, or another known technique. The average diameter of the beads is a volume-based median diameter measured by a particle size distribution measurement device employing a laser diffraction and scattering method. Examples of such a particle size distribution measurement device include "MICROTRAC MT3000II" manufactured by NIKKISO CO., LTD.

The beads are insoluble or substantially insoluble in a solvent or a solution used in measurement. The beads may be porous, substantially porous, hollow, partially hollow, or the like. The beads are preferably non-porous solids or substantially non-porous solids (e.g., essentially pore-free solids). The beads may substantially absorb or absorb the solvent. Preferably, the beads do not absorb or do not substantially absorb the solvent.

A material of the beads can be selected from: plastics and synthetic polymers (e.g., polyethylene, polypropylene, polystyrene, polyamide, polyurethane, phenolic polymer, nitrocellulose, and the like); naturally derived polymers (latex rubber, polysaccharide-supported metal, and metal compounds (e.g., metal compounds including gold, silver, steel, aluminum, copper, and the like)); inorganic glass; silica; mixtures thereof; and the like. The beads preferably include a magnetic material. In addition, the beads is preferably capable of being optically detected.

The beads may be commercially available beads. For example, carboxyl-functionalized paramagnetic beads (diameter: 2.7 pm) manufactured by Agilent Technologies, Inc. can be used.

In terms of, for example, stabilization of an antigenantibody reaction, a buffer solution (buffer) used for the beads is preferably a buffer solution to which a chelating agent is added, and more preferably a buffer solution to which EDTA is added. Addition of EDTA makes it possible to suppress a measurement error occurrence rate. In terms of suppression of the measurement error occurrence rate (in particular, the measurement error occurrence rate resulting from the measurement becoming impossible due to aggregation of the beads), the chelating agent is preferably added to the buffer solution used for the capture beads.

In this specification, the capture beads are beads to each of which the complex including TDP-43 can be immobilized. For example, the capture beads can be obtained by immobilizing, directly or indirectly to each bead, a molecule which can bind to TDP-43 (hereinafter, also referred to as "capture molecule"). As the capture molecule, the capture antibody described above is exemplified.

A method for preparing the capture beads is not limited, provided that the capture molecule can be bound to each of the beads. For example, the capture beads may be prepared with use of a commercially available kit such as Simoa (trademark) Homebrew Assay Development kit manufactured by Quanterix Corporation. In order to suppress non-specific binding, the capture beads may be blocked with bovine serum albumin (BSA) or the like.

In this specification, the non-capture beads are beads each of which does not substantially bind to TDP-43. The non-capture beads each do not have the capture molecule. A material of the beads used as the non-capture beads may be the same as or different from that of the beads used for the capture beads. As the non-capture beads, Dye-Encoded Helper Beads manufactured by Quanterix Corporation may be, for example, used. In order to suppress non-specific binding, the non-capture beads may be blocked with bovine serum albumin (BSA) or the like.

A method for forming the immune complex of TDP-43 in the biological sample, the capture antibody, and the detection antibody on each of the capture beads is not limited, provided that the present embodiment is realized. For example, the immune complex can be formed as follows. First, TDP-43 in the biological sample is bound to the capture antibody so that TDP-43 is bound to each of the capture beads (preferably, TDP-43 is captured). Next, the detection antibody is bound to TDP-43 captured by the capture antibody. The immune complex is thus formed.

Alternatively, as the method for forming the immune complex, the immune complex of TDP-43 in the biological sample, the capture antibody, and the detection antibody may be formed in a buffer solution, and then the immune complex may be bound to each of the capture beads. For example, the beads to each of which avidin or streptavidin is immobilized are brought into contact with the complex of the biotin-labeled capture antibody, TDP-43, and the detection antibody. This allows the immune complex to be immobilized on each of the capture beads.

The immune complex is formed in the presence of the non-capture beads. A ratio of the number of the non-capture beads to the number of the capture beads when the measurement sample is prepared is preferably such that the non-capture beads are not less than 2.3 and more preferably not less than 3 in a case where the capture beads are regarded as 1. The ratio of the number of the non-capture beads to the number of the capture beads is preferably such that the non-capture beads are not more than 9 in a case where the capture beads are regarded as 1. In a case where the ratio of the number of the non-capture beads to the number of the capture beads falls within the above range, it is possible to detect TDP-43 more sensitively.

The number of the entire beads (the capture beads and the non-capture beads) mixed with the biological sample or the diluted biological sample may be, for example, not less than 400,000 with respect to 90 pL of the measurement sample. The number of the entire beads (the capture beads and the non-capture beads) may be not more than 500,000 with respect to 90 pL of the measurement sample.

Preparation of the measurement sample may be carried out in a buffer solution (e.g., 1 to 5x PBS) in which the immune complex can be formed. During preparation of the measurement sample, the capture beads may be washed with a buffer solution after TDP-43 in the biological sample is captured by the capture antibody and before the detection antibody is bound. The capture beads may also be washed with a buffer solution after the immune complex of TDP-43 in the biological sample, the detection antibody, and the capture antibody is formed on each of the capture beads.

The detection antibody may be added so that a final concentration thereof becomes 0.05 pg/mL to 1 pg/mL (preferably, approximately 0.1 pg/mL to 0.5 pg/mL) with respect to 100 pL of the biological sample or the diluted biological sample. The detection antibody preferably has the labeling substance.

### (Detecting step)

In the detecting step, a signal derived from the immune complex in the measurement sample is detected.

Detection of the signal derived from the immune complex in the measurement sample can be carried out by detecting the labeling substance included in the immune complex. The labeling substance is not particularly limited, provided that the signal which can be detected is generated. The labeling substance may be, for example, a substance which itself generates the signal (hereinafter, also referred to as "signal generating substance") or may be a substance which causes the signal to be generated by catalyzing a reaction of another substance. Examples of the signal generating substance include fluorescent substances and radioactive isotopes. Examples of the substance which causes the signal which can be detected to be generated by catalyzing a reaction of another substance include enzymes. Examples of the enzymes include alkaline phosphatase, peroxidase, β-galactosidase, and luciferase. Examples of the fluorescent substances include fluorescent dyes such as fluorescein isothiocyanate (FITC), rhodamine, and Alexa Fluor (registered trademark) and fluorescent proteins such as GFP. Examples of the radioactive isotopes include 1251, 14C, and 32P. Among them, as the labeling substance, an enzyme is preferable, and β-galactosidase is particularly preferable.

A substrate of the enzyme can be selected, as appropriate, from known substrates in accordance with the type of the enzyme. In a case where alkaline phosphatase is used as the enzyme, examples of the substrate include: chemiluminescent substrates such as CDP-Star (registered trademark) (4-chloro-3-(methoxyspiro[1,2-dioxetan-3,2'-(5'-chloro) tricyclo [3.3.1.13,7]decane]-4-yl)disodium phenylphosphate) and CSPD (registered trademark) (3-(4-methoxyspiro[1,2-dioxetan-3,2-(5'-chloro) tricyclo[3.3.1.13,7]decane]-4-yl)disodium phenylphosphate); and chromogenic substrates such as 5-bromo-4-chloro-3-indolyl phosphate (BCIP), 5-bromo-6-chloro-indolyl phosphate disodium, and p-nitrophenyl phosphate. In a case where β-galactosidase is used as the enzyme, resorufin β-D-galactopyranoside can be used. In a case where alkaline phosphatase is used as the enzyme, examples of the substrate include: chemiluminescent substrates such as CDP-Star (registered trademark) (4-chloro-3-(methoxyspiro[1,2-dioxetan-3,2'-(5'-chloro) tricyclo [3.3.1.13,7]decane]-4-yl)disodium phenylphosphate) and CSPD (registered trademark) (3-(4-methoxyspiro[1,2-dioxetan-3,2-(5'-chloro) tricyclo [3.3.1.13,7]decane]-4-yl)disodium phenylphosphate); and chromogenic substrates such as 5-bromo-4-chloro-3-indolyl phosphate (BCIP), 5-bromo-6-chloro-indolyl phosphate disodium, and p-nitrophenyl phosphate. In a case where β-galactosidase is used as the enzyme, resorufin β-D-galactopyranoside can be used. In cases where peroxidase is used as the enzyme, Super Signal ELISA series manufactured by Thermo Fisher SCIENCE Inc. may be, for example, used. These enzyme reactions are preferably carried out before the beads are placed in microwells described later, but may be carried out after the beads are placed in the microwells.

The detection antibody may be biotinylated, and then bound to avidin or streptavidin each of which is labeled with the labeling substance. Thereafter, the signal may be detected.

Detection of the signal can be carried out with respect to the whole or a part of the beads contained in the measurement sample and including the capture beads and the non-capture beads. Detection of the signal is preferably carried out with respect to each bead. Note, here, that the capture beads differ from the non-capture beads in the presence or absence, intensity, wavelength, and the like of the signal. Therefore, it is preferable to use a detection device which can discriminate these.

As a method for detecting the signal of each bead, a flow cytometer may be, for example, used to detect the signal, in a case where the signal is fluorescence. Alternatively, the measurement sample may be brought into contact with a substrate having a plurality of microwells, and the capture beads and the non-capture beads in the measurement sample may be placed in the microwells. Then, the signal in each microwell may be detected.

On the substrate, the microwells can be arranged on a substantially flat surface or in a non-planar three-dimensional array. The microwells may be arranged in a regular pattern or may be randomly distributed. The microwells are preferably a regular pattern of parts on the substantially flat surface.

The number of the microwells included in the substrate may be approximately 1,000 to 1,000,000, preferably 10,000 to 500,000, and more preferably approximately 100,000 to 500,000, per measurement sample.

Examples of a material of the substrate include: glass (including modified and/or functionalized glass); plastics (polypropylene, polyethylene, polybutylene, polyurethane, cyclic olefin copolymer (COC), cyclic olefin polymer (COP), Teflon (registered trademark), polysaccharide, nylon, nitrocellulose, and the like); elastomers (e.g., dimethylsiloxane, polyurethane, and the like); composite materials; ceramic; silica and silica-based materials (including silicon and modified silicon); carbon; metal; and fiber optic bundles.

A volume of each microwell may be set as appropriate, in accordance with the size of the beads. For example, the number of the beads held in a single well is approximately 1 to 5, preferably approximately 1 to 2, and more preferably 1. Therefore, the volume of each microwell is preferably such that the above number of the beads are held in a single well (e.g., Specification of US Patent Application Publication No. 2011/0212848, International Publication No. WO 2011/109364). More specifically, the volume of each microwell falls within a range of 100 aL to 1 pL, preferably 1 fL to 500 fL, and more preferably 10 fL to 90 fL. The plurality of microwells present on a single substrate each preferably have the substantially same volume.

A method for forming the microwells is not limited, provided that the microwells having a desired size can be formed. For example, the microwells may be formed by a known method such as photolithography, a stamping technique, a molding technique, or an etching technique.

Placement of the beads in the microwells can be carried out with use of a known method. The beads may be placed in all of the microwells on the substrate or may be placed in a part of the microwells.

For example, the measurement sample may be brought into contact with the substrate on which the microwells are designed, and then the substrate and the solution are centrifuged so that the beads are placed in the microwells. In a case where the beads are magnetic, a magnet may be used to place the beads in the microwells. In a case where the microwells are formed at ends of a fiber optic bundle, the measurement sample may be brought into contact with the other ends and their vicinities of the optical fibers, and then a force such as a centrifugal force or a pressure may be applied so that the beads are placed in the microwells.

After the beads are placed in the microwells, the measurement sample remaining on the substrate around the microwells may be removed, and a surface of the substrate may be washed and/or wiped out so as to remove any extra measurement sample. As the substrate including the microwells, Simoa (trademark) Disc manufactured by Quanterix Corporation may be, for example, used.

The microwells after the beads are placed therein may be sealed with a fluorous liquid, an oil (e.g., a mineral oil or a fluorinated oil), a ferromagnetic fluid, a nonaqueous polymer solution (e.g., a thickener), or the like. For example, Simoa (trademark) Sealing Oil manufactured by Quanterix Corporation may be used for sealing.

In a case where the labeling substance is an enzyme or a fluorescent dye, detection of the beads placed in the microwells may be carried out, for example, by capturing an image of the signal with use of a CCD camera or the like. In a case where the labeling substance is a radioactive isotope, detection may be carried out with use of autoradiography.

The beads placed in the microwells are the capture beads or the non-capture beads. The capture beads which have captured TDP-43 each emit the signal derived from the labeling substance. (i.e., the signal derived from the immune complex). On the contrary, the beads which have not captured the target protein and the non-capture beads each do not emit the signal derived from the labeling substance. For this reason, the target protein can be quantified by counting the number of wells which each emit the signal derived from the labeling substance. The microwells in which the signal is detected may be all of the microwells on the substrate or may be a part of the microwells.

The number of the wells which each emit the signal derived from the labeling substance can be measured, for example, with use of Simoa (trademark) HD-1 Analyzer and Simoa (trademark) HD-X Analyzer each manufactured by Quanterix Corporation. In a case where Simoa (trademark) HD-1 Analyzer and Simoa (trademark) HD-X Analyzer are used, the intensity of the signal is expressed as an average enzymes per bead (AEB) value. For this reason, the AEB value may be used as the intensity of the signal derived from the immune complex in the measurement sample.

Further, a calibration curve may be used to convert the AEB value to a measured value of TDP-43. Note, here, that the measured value of TDP-43 refers to a value reflecting the amount or the concentration of TDP-43. In a case where the measured value is expressed by the "amount", the measured value may be a mole or a mass, but is preferably expressed by a mass. In a case where the value is expressed by the "concentration", the measured value may be a molar concentration or may be a ratio of a mass to a constant volume of the biological sample (mass/volume), but is preferably mass/volume.

In the measurement method in accordance with an embodiment of the present invention, it is preferable to use β-galactosidase as the labeling substance and resorufin β-D-galactopyranoside as the substrate, in accordance with the protocol by the Quanterix Corporation. Further, it is preferable that the beads are applied to Simoa (trademark) Disc and the intensity of the signal is obtained with use of Simoa (trademark) HD-1 Analyzer and Simoa (trademark) HD-X Analyzer.

An aspect of the measurement method in accordance with an embodiment of the present invention is a sandwich immunoassay method in which a sandwich immune complex containing TDP-43 is formed and TDP-43 is detected.

By measuring a neurofilament light chain (NfL) in the biological sample in addition to measuring TDP-43 in the biological sample by the measurement method in accordance with an embodiment of the present invention, it is possible to more improve the accuracy of information on whether or not the subject is affected by TDP-43 proteinopathy.

By the measurement method in accordance with an embodiment of the present invention, it is possible to quantify TDP-43, even in a case where the concentration of TDP-43 contained in the measurement sample is not more than 6.65 pg/mL, as shown in Examples. In the measurement method in accordance with an embodiment of the present invention, it is also possible to quantify TDP-43, even in a case where the concentration of TDP-43 in the measurement sample is not more than 6 pg/mL, not more than 5 pg/ml, not more than 4 pg/ml, not more than 3 pg/ml, or not more than 2 pg/ml. The lower limit of the concentration of TDP-43 contained in the measurement sample is not particularly limited. In terms of ease of quantification, the concentration of TDP-43 in the measurement sample is preferably not less than 1 pg/mL, and may be more preferably not less than 1.3 pg/mL or not more than 1.4 pg/mL.

### [2. Method for obtaining information on whether or not subject is affected by TDP-43 proteinopathy]

The present invention also relates to a method for obtaining information on whether or not a subject is affected by TDP-43 proteinopathy (hereinafter, may be abbreviated to "information obtaining method"). In the information obtaining method in accordance with an embodiment of the present invention, the information on whether or not the subject is affected by TDP-43 proteinopathy is obtained on the basis of an intensity of a signal obtained by [1. Method for measuring TDP-43]. Examples of TDP-43 proteinopathy include amyotrophic lateral sclerosis (ALS) and frontotemporal lobar degeneration (FTLD).

More specifically, the intensity of the signal derived from TDP-43 in a biological sample of the subject is compared with a given reference value. Then, in a case where the intensity of the signal derived from TDP-43 in the biological sample of the subject is higher than the given reference value, information that the subject is affected by TDP-43 proteinopathy is obtained.

Note, here, the given reference value refers to a reference value of the intensity of the signal derived from TDP-43. The reference value may be determined on the basis of an intensity of a signal derived from TDP-43 in a biological sample of a subject who does not develop TDP-43 proteinopathy (negative control value). Alternatively, the reference value may be determined on the basis of an intensity of a signal derived from TDP-43 in a biological sample of a subject who has developed TDP-43 proteinopathy (positive control value). The reference value may be determined on the basis of both of the negative control value and the positive control value. For example, a plurality of positive control values and a plurality of negative control values may be obtained, and then a value which allows the most accurate classification of positive and negative may be used as the reference value on the basis of these plurality of values. Note, here, that the value which allows the most accurate classification can be set as appropriate on the basis of an index such as sensitivity, specificity, a positive predictive value, or a negative predictive value in accordance with a purpose of an inspection. The reference value which allows the most accurate classification of positive and negative may be set from the viewpoint of the sensitivity, the specificity, the negative predictive value, the positive predictive value, or the like.

Instead of the intensity of the signal, a measured value of TDP-43 may be used to obtain the information on whether or not the subject is affected by TDP-43 proteinopathy.

### [3. Device which measures TDP-43]

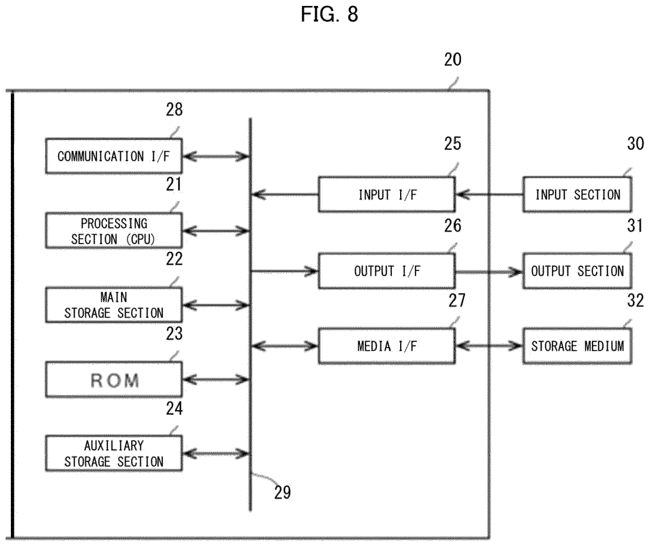

The present invention also relates to a device which measures TDP-43 (hereinafter, may be abbreviated to "measurement device"). The measurement device in accordance with an embodiment of the present invention is described in detail with reference to Fig. 8. Fig. 8 is a view illustrating a schematic configuration of a measurement device 20 in accordance with an embodiment of the present invention. The measurement device 20 is a device which measures TDP-43 in a biological sample collected from a subject.

As illustrated in Fig. 8, the measurement device 20 is connected to an input section 30, an output section 31, and a storage medium 32. In the measurement device 20, a processing section (CPU) 21, a main storage section 22, a read only memory (ROM) 23, an auxiliary storage section 24, a communication interface (I/F) 28, an input interface (I/F) 25, an output interface (I/F) 26, and a media interface (I/F) 27 are connected to each other by a bus 29 so that data communication can be performed therebetween.

The CPU 21 is a processing section of the measurement device 20. The measurement device 20 functions by the CPU 21 executing a computer program stored in the auxiliary storage section 24 or the ROM 23 and processing obtained data.

The ROM 23 is constituted by a mask ROM, a PROM, an EPROM, an EEPROM (registered trademark), or the like, and a computer program executed by the CPU 21 and data used for the execution are recorded in the ROM 23. The CPU 21 may be an MPU 21. In the ROM 23, a boot program executed by the CPU 21 at a time of start of the measurement device 20, a program relating to operation of hardware of the measurement device 20, and settings are stored.

The main storage section 22 is constituted by a random access memory (RAM) such as an SRAM or a DRAM. The main storage section 22 is used for reading of computer programs recorded in the ROM 23 and the auxiliary storage section 24. The main storage section 22 is also used as a working area in which the CPU 21 executes these computer programs.

The auxiliary storage section 24 is constituted by a hard disk, a semiconductor memory device such a flash memory, an optical disk, or the like. In the auxiliary storage section 24, stored are (i) various computer programs which are executed by the CPU 21, such as an operating system and an application program, and (ii) various setting data used for execution of the computer programs. Specifically, in the auxiliary storage section 24, stored are a measurement program (described later), a reference value of an intensity of a signal, a reference value of a measured value of phosphorylated tau protein, and the like.

The communication I/F 28 is constituted by a serial interface such as a USB, an IEEE1394, or an RS-232C, a parallel interface such as an SCSI, an IDE, or an IEEE1284, an analog interface which is constituted by a D/A converter, an A/D converter, or the like, a network interface controller (NIC), or the like. Under control by the CPU 21, the communication I/F 28 receives data from a measurement unit or another external device, and transmits or displays, to or on the measurement unit or the outside, information stored or generated by the measurement device 20, as necessary. The communication I/F 28 may perform communication with the measurement unit or another external device via a network.

The input I/F 25 is constituted by, for example, a serial interface such as a USB, an IEEE1394, or an RS-232C, a parallel interface such as an SCSI, an IDE, or an IEEE1284, an analog interface which is constituted by a D/A converter, an A/D converter, or the like, or the like. The input I/F 25 accepts a character input, a click, a voice input, or the like performed by the input section 30. A content of an accepted input is stored in the main storage section 22 or the auxiliary storage section 24.

The input section 30 is constituted by a touch panel, a keyboard, a mouse, a pen tablet, a microphone, or the like, and performs a character input or a voice input into the measurement device 20. The input section 30 may be connected to the measurement device 20 from the outside of the measurement device 20, or may be integrated with the measurement device 20.

The output I/F 26 is constituted by, for example, an interface similar to that constituting the input I/F 25. The output I/F 26 outputs, to the output section 31, information generated by the CPU 21. The output I/F 26 outputs, to the output section 31, information generated by the CPU 21 and stored in the auxiliary storage section 24.

The output section 31 is constituted by, for example, a display, a printer, or the like, and displays a measurement result transmitted from the measurement unit and various operating windows, a measurement result, or the like in the measurement device 20.

The media I/F 27 reads, for example, application software or the like stored in the storage medium 32. The read application software or the like is stored in the main storage section 22 or the auxiliary storage section 24. The media I/F 27 also writes, in the storage medium 32, information generated by the CPU 21. The media I/F 27 writes, in the storage medium 32, information generated by the CPU 21 and stored in the auxiliary storage section 24.

The storage medium 32 is constituted by a flexible disk, a CD-ROM, a DVD-ROM, or the like. The storage medium 32 is connected to the media I/F 27 by a flexible disk drive, a CD-ROM drive, a DVD-ROM drive, or the like. In the storage medium 32, for example, an application program for a computer to execute operation may be stored.

The CPU 21 may obtain application software and various settings necessary to control the measurement device 20, via a network instead of reading from the ROM 23 or the auxiliary storage section 24. The application program can be stored in the auxiliary storage section 24 of a server computer on a network, and the measurement device 20 can access the server computer, download the computer program, and store the computer program in the ROM 23 or the auxiliary storage section 24.

In the ROM 23 or the auxiliary storage section 24, for example, an operating system which provides a graphical user interface environment, such as Windows (registered trademark) manufactured and sold by Microsoft Corporation in America, may be installed.

The processing section 21 accepts an input which is performed by an inspector on the input section 30 and which is for obtaining a signal derived from an immune complex with regard to a measurement sample that includes (i) non-capture beads and (ii) capture beads to each of which the immune complex binds. Next, the processing section 21 obtains the signal which is derived from the immune complex and which has been detected by the measurement unit (described later) or the like. The processing section 21 then stores, in the auxiliary storage section 24 or the like, an intensity of the signal thus obtained.

The measurement device 20 can be a personal computer or the like. The measurement device 20 may be embedded in the measurement unit (described later).

### [4. System which measures TDP-43]

The present invention also relates to a system which measures TDP-43. The system which measures TDP-43 includes the measurement device 20 and the measurement unit. The system which measures TDP-43 is a system which measures TDP-43 in a biological sample collected from a subject.

The measurement unit includes at least a detecting section including an image capturing device for detecting a signal, such as a CCD camera. In some cases, the measurement unit may further include a sample preparing section for preparing a measurement sample. The detecting section may be a flow cytometer. The detecting section may include a pressurizer, a decompression device, or a centrifuge for placing, in microwells, beads in the measurement sample.

The measurement unit may be integrated with the measurement device 20. Examples of the device which includes the detecting section including the image capturing device and which is integrated with the measurement device 20 include Simoa (trademark) HD-1 Analyzer and Simoa (trademark) HD-X Analyzer.

### [5. Device which obtains information on whether or not subject is affected by TDP-43 proteinopathy]

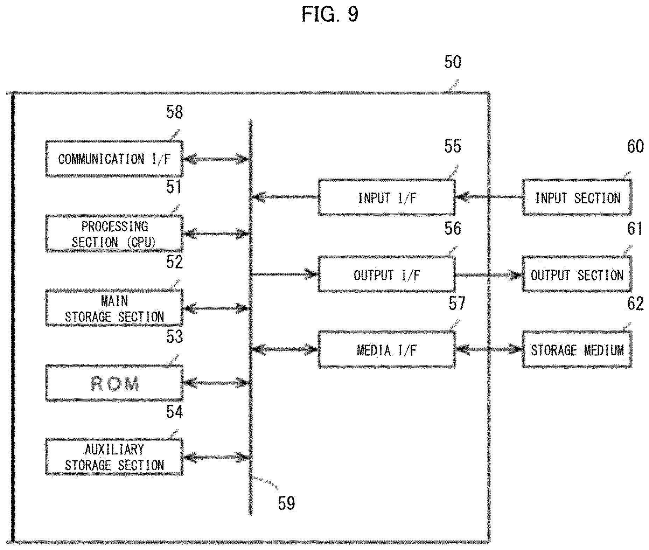

The present invention also relates to a device which obtains information on whether or not a subject is affected by TDP-43 proteinopathy (hereinafter, may be abbreviated to "information obtaining device"). Fig. 9 illustrates the information obtaining device in accordance with an embodiment of the present invention. The information obtaining device 50 is a device which obtains the information on whether or not the subject has TDP-43 proteinopathy, on the basis of an intensity of a signal of TDP-43 in a biological sample collected from the subject.

As illustrated in Fig. 9, the information obtaining device 50 is connected to an input section 60, an output section 61, and a storage medium 62. In the information obtaining device 50, a processing section (CPU) 51, a main storage section 52, a read only memory (ROM) 53, an auxiliary storage section 54, a communication interface (I/F) 58, an input interface (I/F) 55, an output interface (I/F) 56, and a media interface (I/F) 57 are connected to each other by a bus 59 so that data communication can be performed therebetween.

Details of each configuration in Fig. 9 are similar to those of each configuration in Fig. 8.

The processing section 51 accepts an input which is performed by an inspector on the input section 60 and which is for obtaining the signal derived from an immune complex with regard to a measurement sample that includes (i) non-capture beads and (ii) capture beads to each of which the immune complex binds. Next, the processing section 21 obtains the signal which is derived from the immune complex and which has been detected by the measurement unit (described later) or the like. Subsequently, an intensity of the obtained signal is compared with a reference value of the intensity of the signal which reference value is stored in the auxiliary storage section 54 or the like. In a case where the intensity of the signal with regard to the measurement sample is higher than the reference value, the processing section 51 determines that there is a possibility that the subject is affected by TDP-43 proteinopathy. In a case where the intensity of the signal obtained with regard to the measurement sample is lower than the reference value, the processing section 51 determines that there is no possibility that the subject is affected by TDP-43 proteinopathy. The processing section 51 stores, in the auxiliary storage section 54 or the like, these determination results as information on whether or not the subject has TDP-43 proteinopathy. The processing section 51 may output these results to the output section 61 or store these results in the storage medium 62.

### [6. System which obtains information on whether or not subject is affected by TDP-43 proteinopathy]

The present invention also relates to a system which obtains information on whether or not a subject is affected by TDP-43 proteinopathy (hereinafter, may be abbreviated to "information obtaining system"). The information obtaining system includes the information obtaining device 50 and the measurement unit. The information obtaining system is a system which measures TDP-43 in a biological sample collected from the subject. Details of the measurement unit are similar to those of the measurement unit described in [4. System which measures TDP-43].

The measurement unit may be integrated with the information obtaining device 50. Examples of the device which includes the detecting section including the image capturing device and which is integrated with the information obtaining device 50 include Simoa (trademark) HD-1 Analyzer and Simoa (trademark) HD-X Analyzer.

### [7. Computer program for measuring TDP-43]

The present invention also relates to a computer program for measuring TDP-43 (hereinafter, may be abbreviated to "measurement program"). The measurement program is used as a measurement program for controlling operation of the measurement device 20. The measurement program causes a computer to carry out a step of obtaining a signal derived from an immune complex with regard to a measurement sample that includes (i) non-capture beads and (ii) capture beads to each of which the immune complex binds.

### [8. Computer program for obtaining information on whether or not subject is affected by TDP-43 proteinopathy]

The present invention also relates to a computer program for obtaining information on whether or not a subject is affected by TDP-43 proteinopathy (hereinafter, may be abbreviated to "information obtaining program"). The information obtaining program is used as a measurement program for controlling operation of the information obtaining device 50. The information obtaining program causes a computer to carry out a step of obtaining a signal derived from an immune complex with regard to a measurement sample that includes (i) non-capture beads and (ii) capture beads to each of which the immune complex binds.

The measurement program and the information obtaining program may be stored in a storage medium such as a hard disk, a semiconductor memory device such as a flash memory, or an optical disk. A form in which the programs are stored in the storage medium is not limited, provided that the information obtaining device can read the programs. Storage in the storage medium is preferably nonvolatile.

### [9. Reagent and reagent kit for detecting TDP-43]

The present invention also relates to a reagent for detecting TDP-43 (hereinafter, may be abbreviated to "reagent for detection"). The reagent for detection in accordance with an embodiment of the present invention is used in [1. Method for measuring TDP-43], and includes capture beads and non-capture beads. A preferable ratio of the number of the non-capture beads to the number of the capture beads in the reagent for detection is as described in [1. Method for measuring TDP-43].

The present invention also relates to a reagent kit for detecting TDP-43 (hereinafter, may be abbreviated to "reagent kit"). The reagent kit is used in the measurement method in accordance with an embodiment of the present invention, and includes the above reagent for detection.

The reagent kit may further include a detection antibody, and may include another reagent, an instrument, and the like. In a case where a capture antibody is not immobilized to each of the beads, the reagent kit may further include a reagent which contains the capture antibody. In the reagent kit, a plurality of different reagents may be mixed together at respective appropriate volumes and/or in an appropriate form. The reagent kit may provide the plurality of different reagents in respective different vessels. The reagent kit may include instructions which show a procedure for measuring TDP-43. The instructions may be written or printed on paper or another medium. Alternatively, the instructions may be written in a magnetic tape or an electronic medium which is readable by a computer or the like, such as a disk or a CD-ROM.

### [10. Method for diagnosing TDP-43 proteinopathy]

The present invention also relates to a method for diagnosing TDP-43 proteinopathy (hereinafter, may be abbreviated to "diagnosing method"). The diagnosing method in accordance with an embodiment of the present invention includes a diagnosing step of diagnosing whether or not a subject is affected by TDP-43 proteinopathy, on the basis of an intensity of a signal obtained by [1. Method for measuring TDP-43]. The diagnosing method in accordance with an embodiment of the present invention encompasses a diagnosis by a physician or another qualified medical expert.

In the above diagnosing step, the intensity of the signal derived from TDP-43 in a biological sample of the subject is compared with a given reference value. Then, in a case where the intensity of the signal derived from the TDP-43 in the biological sample of the subject is higher than the given reference value, the subject is diagnosed as being affected by TDP-43 proteinopathy. The "given reference value" is as described in [2. Method for obtaining information on whether or not subject is affected by TDP-43 proteinopathy].

Instead of the intensity of the signal, a measured value of TDP-43 may be used to diagnose whether or not the subject is affected by TDP-43 proteinopathy.

By diagnosing whether or not the subject is affected by TDP-43 proteinopathy, it is possible to objectively observe and evaluate progression of the disease, progression of treatment, or an effect of the treatment.

### [11. Method for aiding diagnosis of patient]

The present invention also relates to a method for aiding a diagnosis of a patient. The method for aiding a diagnosis of a patient in accordance with an embodiment of the present invention includes a step of obtaining data suggesting whether or not a biological sample is a biological sample derived from a patient affected by TDP-43 proteinopathy, on the basis of an intensity of a signal obtained by [1. Method for measuring TDP-43].

### [12. Summary of embodiments]

The embodiments of the present invention include, for example, aspects described below.
<1> A method for measuring TDP-43 in a biological sample, the method including: a preparing step of preparing a measurement sample including (i) non-capture beads and (ii) capture beads to each of which an immune complex of TDP-43 in a biological sample, a capture antibody, and a detection antibody binds, by forming the immune complex on each of the capture beads in the presence of the non-capture beads; and a detecting step of detecting a signal derived from the immune complex in the measurement sample, wherein each of the non-capture beads does not bind to the immune complex, at least one of the detection antibody and the capture antibody specifically recognizes the TDP-43, an epitope of the capture antibody and an epitope of the detection antibody differ from each other, a ratio of the number of the non-capture beads to the number of the capture beads is such that the non-capture beads are not less than 2.3 and not more than 9 in a case where the capture beads are regarded as 1, and a concentration of the capture beads and the non-capture beads is not less than 400,000 beads and not more than 500,000 beads with respect to 90 pL of the measurement sample.
<2> A method for measuring TDP-43 in a biological sample, the method including: a preparing step of preparing a measurement sample including (i) non-capture beads and (ii) capture beads to each of which an immune complex of TDP-43 in a biological sample, a capture antibody, and a detection antibody binds, by forming the immune complex on each of the capture beads in the presence of the non-capture beads; and a detecting step of detecting a signal derived from the immune complex in the measurement sample, wherein each of the non-capture beads does not bind to the immune complex, at least one of the detection antibody and the capture antibody specifically recognizes the TDP-43, an epitope of the capture antibody and an epitope of the detection antibody differ from each other, a ratio of the number of the non-capture beads to the number of the capture beads is such that the non-capture beads are not less than 2.3 and not more than 9 in a case where the capture beads are regarded as 1, a concentration of the capture beads and the non-capture beads is not less than 400,000 beads and not more than 500,000 beads with respect to 90 pL of the measurement sample, and EDTA is added to a buffer solution used for the capture beads and the non-capture beads.
<3> The method described in < 1> or <2>, wherein the TDP-43 is phosphorylated TDP-43.
<4> The method described in any one of <1> through <3>, wherein a concentration of the TDP-43 contained in the measurement sample is not less than 1.43 pg/mL and not more than 6.65 pg/mL.
<5> The method described in any one of <1> through <4>, wherein one of the epitope of the detection antibody and the epitope of the capture antibody includes a peptide composed of the 203rd to 209th amino acids in an amino acid sequence represented by SEQ ID NO. 1, and the other of the epitope of the detection antibody and the epitope of the capture antibody is (i) an epitope included in a peptide composed of the 261st to 414th amino acids in the amino acid sequence represented by SEQ ID NO. 1 or (ii) an epitope including a peptide which is composed of the 404th to 410th amino acids in the amino acid sequence represented by SEQ ID NO. 1 and in which the 409th and 410th serines are phosphorylated.
<6> The method described in any one of <1> through <5>, wherein in the detecting step, the measurement sample is brought into contact with a substrate having a plurality of microwells, the capture beads and the non-capture beads in the measurement sample are respectively placed in the plurality of microwells, the signal derived from the immune complex on each of the capture beads placed in corresponding ones of the plurality of microwells is detected, and each of the plurality of microwells has a volume which allows merely one of the capture beads or the non-capture beads to be held in the each of the plurality of microwells.
<7> The method described in any one of <1> through <6>, wherein, in the detecting step, detection of the signal is carried out by capturing an image of the signal derived from the immune complex.
<8> The method described in any one of <1> through <7>, wherein the biological sample is spinal fluid or a blood sample.
<9> The method described in <8>, wherein the blood sample is blood serum or blood plasma.
<10> The method described in any one of <1> through <9>, wherein the immune complex is a complex of the capture antibody, the TDP-43 in the biological sample, and the detection antibody, and the capture antibody binds to each of the capture beads.
<11> The method described in any one of <1> through <10>, wherein the immune complex is formed by mixing the capture antibody which binds to each of the capture beads, the biological sample which contains the TDP-43, and the detection antibody in the presence of the non-capture beads.
<12> A method for obtaining information on whether or not a subject is affected by TDP-43 proteinopathy, on the basis of an intensity of a signal obtained by the method described in any one of <1> through <11>.
<13> A reagent or reagent kit used in the method described in any one of <1> through <12>, the reagent or reagent kit including the capture beads and the non-capture beads.
<14> A device which measures TDP-43 in a biological sample collected from a subject, the device including a processing section, wherein the processing section obtains a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads, each of the non-capture beads does not bind to the immune complex, at least one of the detection antibody and the capture antibody specifically recognizes the TDP-43, an epitope of the capture antibody and an epitope of the detection antibody differ from each other, a ratio of the number of the non-capture beads to the number of the capture beads is such that the non-capture beads are not less than 2.3 and not more than 9 in a case where the capture beads are regarded as 1, and a concentration of the capture beads and the non-capture beads is not less than 400,000 beads and not more than 500,000 beads with respect to 90 pL of the measurement sample.
<15> A device which obtains information on whether or not a subject has TDP-43 proteinopathy, on the basis of an intensity of a signal derived from TDP-43 in a biological sample collected from the subject, the device including a processing section, wherein the processing section obtains a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads, each of the non-capture beads does not bind to the immune complex, at least one of the detection antibody and the capture antibody specifically recognizes the TDP-43, an epitope of the capture antibody and an epitope of the detection antibody differ from each other, the processing device obtains information on whether or not a subject has TDP-43 proteinopathy, on the basis of an intensity of the signal, a ratio of the number of the non-capture beads to the number of the capture beads is such that the non-capture beads are not less than 2.3 and not more than 9 in a case where the capture beads are regarded as 1, and a concentration of the capture beads and the non-capture beads is not less than 400,000 beads and not more than 500,000 beads with respect to 90 pL of the measurement sample.
<16> A computer program for measuring TDP-43, the computer program causing a computer to carry out a step of obtaining a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads, wherein the biological sample is collected from a subject, each of the non-capture beads does not bind to the immune complex, at least one of the detection antibody and the capture antibody specifically recognizes the TDP-43, an epitope of the capture antibody and an epitope of the detection antibody differ from each other, a ratio of the number of the non-capture beads to the number of the capture beads is such that the non-capture beads are not less than 2.3 and not more than 9 in a case where the capture beads are regarded as 1, and a concentration of the capture beads and the non-capture beads is not less than 400,000 beads and not more than 500,000 beads with respect to 90 pL of the measurement sample.
<17> A computer program for obtaining information on whether or not a subject has TDP-43 proteinopathy, the computer program causing a computer to carry out: a step of obtaining a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads; and a step of obtaining information on whether or not a subject from whom the biological sample has been collected has TDP-43 proteinopathy, on the basis of an intensity of the signal, wherein each of the non-capture beads does not bind to the immune complex, at least one of the detection antibody and the capture antibody specifically recognizes the TDP-43, an epitope of the capture antibody and an epitope of the detection antibody differ from each other, a ratio of the number of the non-capture beads to the number of the capture beads is such that the non-capture beads are not less than 2.3 and not more than 9 in a case where the capture beads are regarded as 1, and a concentration of the capture beads and the non-capture beads is not less than 400,000 beads and not more than 500,000 beads with respect to 90 pL of the measurement sample.
<18> A method for diagnosing TDP-43 proteinopathy, the method including a diagnosing step of diagnosing whether or not a subject is affected by TDP-43 proteinopathy, on the basis of an intensity of a signal obtained by the method described in any one of <1> through <11>.
< 19> A method for aiding a diagnosis of a patient, the method including a step of obtaining data suggesting whether or not a biological sample is a biological sample derived from a patient affected by TDP-43 proteinopathy, on the basis of an intensity of a signal obtained by the method described in any one of <1> through <11>.

The following description will discuss, in more detail, an embodiment of the present invention with reference to Examples. Obviously, the present invention is not limited to the following Examples, and it is needless to say that various aspects are employed for details. Furthermore, the present invention is not limited to the above-described embodiments, and various modifications can be made within the scope of the claims. The present invention encompasses, in its technical scope, an embodiment obtained by appropriately combining technical means disclosed in differing embodiments. Further, all of the documents listed in this specification are incorporated herein by reference.

### Examples

### [Materials]

Capture beads were prepared with use of beads of Simoa (trademark) Homebrew Assay Development kit manufactured by Quanterix Corporation and in accordance with a protocol attached thereto. As non-capture beads, the Dye-Encoded Helper Beads manufactured by Quanterix Corporation were used. As a bead buffer, a buffer to which EDTA was added was used. As a capture antibody, 60019-2-Ig (Proteintech Group, Inc.) was used. An antigen peptide of 60019-2-Ig is a peptide which corresponds to the 203rd to 209th amino acids in an amino acid sequence of TDP-43 (amino acid sequence registered as NCBI Reference Sequence NP_031401). As a detection antibody, 12892-1-AP (Proteintech Group, Inc.) was used. Biotinylation of the detection antibody was carried out in accordance with the protocol of the above kit. An antigen peptide of 12892-1-AP is included in a peptide composed of the 261st to 414th amino acids in the amino acid sequence of TDP-43 (amino acid sequence registered as NCBI Reference Sequence NP_031401). 12892-1-AP is a polyclonal antibody. Hereinafter, a method for measuring TDP-43 with use of a combination of 60019-2-Ig (capture antibody) and 12892-1-AP (detection antibody) may be abbreviated to a measurement method of an example.

As a target protein, non-phosphorylated TDP-43 or phosphorylated TDP-43 was used. A human TDP-43 standard peptide was used as a standard peptide, in order to create a standard curve. The target protein or the standard peptide was diluted with use of Sample diluent of Simoa (trademark) Homebrew Assay Development kit.

### [Evaluation Example 1] Measurement of lower limit of detectable concentration of human TDP-43 standard peptide

As a capture antibody, 60019-2-Ig (Proteintech Group, Inc.) was used. As a detection antibody, 12892-1-AP (Proteintech Group, Inc.) was used. Then, a human TDP-43 standard peptide was measured with reference to a protocol attached to Simoa (trademark) Homebrew Assay Development kit. The measurement was carried out with use of Simoa (trademark) Enzyme and Substrate kit, Simoa (trademark) Disk kit, Simoa (trademark) Sealing Oil, System buffer 1, System buffer 2, and Simoa (trademark) HD-1 Analyzer and in accordance with a protocol of the manufacturer. In the measurement, a measurement sample and necessary reagents including capture beads, a biotin-labeled antibody, and non-capture beads were set in Simoa (trademark) HD-1 Analyzer. Then, all measuring steps were carried out with use of Simoa (trademark) HD-1 Analyzer.

In Evaluation Example 1, preparation was carried out so that a ratio of the number of the non-capture beads to the number of the capture beads became 3:1 to 9:1. Further, the preparation was carried out so that a concentration of the entire beads (the capture beads and the non-capture beads) became 400,000 to 500,000 beads with respect to 90 pL of the measurement sample.

The detailed measuring steps are as follows. First, the standard peptide and the capture antibody which was bound to each of the capture beads were bound to each other in the presence of the non-capture beads. Next, by further binding the biotinylated detection antibody to the standard peptide bound to the capture antibody, an immune complex of the capture antibody, the standard peptide, and the biotinylated detection antibody was formed on each of the capture beads.

After formation of the immune complex, the capture beads and β-galactosidase-bound streptavidin were reacted in the presence of the non-capture beads. Then, the capture beads were reacted with a substrate (RGP: resorufin β-D-galactopyranoside) of β-galactosidase in the presence of the non-capture beads. The non-capture beads and the capture beads which had completed reacting with the RGP were applied to Simoa (trademark) Disc. Thereafter, the applied beads were stored in respective wells in an array in which a large number of wells of a femtoliter (fL) unit were arranged. An image of each of the beads stored in the respective wells was captured with use of Simoa (trademark) HD-1 Analyzer, and a fluorescence intensity (AEB) was measured. The target protein was quantified by measuring the number of beads which each had a high fluorescence intensity. A buffer was used as a blank.

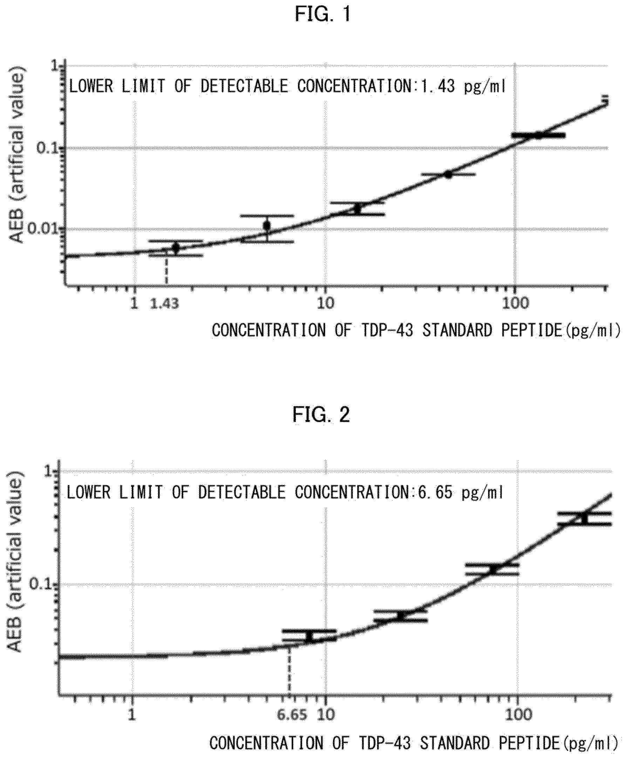

A measurement method in which a capture antibody and a detection antibody of Simoa (trademark) Homebrew Assay Development kit were used was regarded as a measurement method of a comparative example. The measurement method of the comparative example and the measurement method of the example were compared. Fig. 1 shows a measurement result obtained by the measurement method of the example. Fig. 2 shows a measurement result obtained by the measurement method of the comparative example. A horizontal axis in each of Figs. 1 and 2 indicates a concentration (pg/mL) of TDP-43. A vertical axis in each of Figs. 1 and 2 indicates an actually measured value (AEB) obtained by the measurement.

A lower limit of a detectable concentration of the measurement method of the comparison example in Fig. 2 was 6.65 pg/mL. On the contrary, a lower limit of a detectable concentration of the measurement method of the example in Fig. 1 was 1.43 pg/mL. Compared with the measurement method of the comparative example, the measurement method of the example had clearly improved detection sensitivity with respect to a TDP-43 standard peptide.

Moreover, by using, as a bead buffer, a buffer to which EDTA was added, it was possible to stabilize an antigen-antibody reaction.

### [Evaluation Example 2] Quantification of TDP-43 in human cerebrospinal fluid (CSF)

The measurement method of the example and the measurement method of the comparative example were compared with use of human CSF as samples. In the measurement method of the example, a concentration of entire beads and a ratio of the number of non-capture beads to the number of capture beads were set similarly to those in Evaluation Example 1. Non-phosphorylated TDP-43 in the CSF of four patients with amyotrophic lateral sclerosis (ALS), in which abnormally aggregated TDP-43 accumulates within a central nervous system, and four control patients (CTRL) was measured. The CSF was diluted four-fold with Sample diluent of Simoa (trademark) Homebrew Assay Development kit, and then measurement was carried out. A buffer was used as a blank.

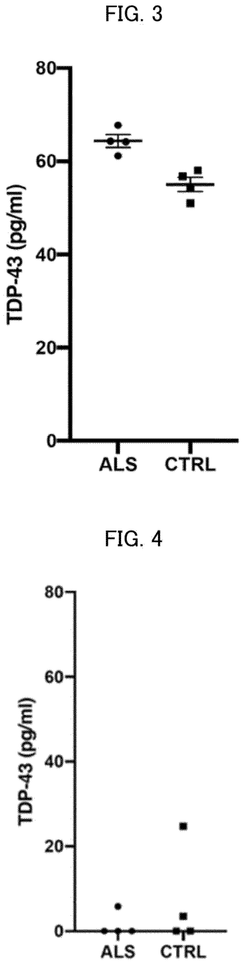

Fig. 3 shows measurement results obtained by the measurement method of the example. Fig. 4 shows measurement results obtained by the measurement method of the comparative example. A vertical axis in each of Figs. 3 and 4 indicates concentrations (pg/mL) of TDP-43 in the CSF.

The CSF samples of the same eight patients were measured by each of the measurement method of the comparative example and the measurement method of the example. As shown in Fig. 4, in the measurement method of the comparative example, measured values of TDP-43 in the samples of seven out of the eight patients were equal to or lower than a lower limit of detection sensitivity (6.65 pg/mL). Furthermore, no measured values were obtained in the samples of five patients (shown as 0 in Fig. 4). Moreover, in the measurement method of the comparative example, there was no clear difference between the ALS patients and the control patients (CTRL) in terms of the barely measured concentrations of TDP-43.

On the contrary, as shown in Fig. 3, in the measurement method of the example, it was possible to measure the concentrations of TDP-43 in all of the CSF samples. A clear difference was also found between the ALS patients and the control patients (CTRL) in terms of the concentrations of TDP-43 in the CSF.

It was found from Evaluation Example 2 that the measurement method of the example enabled quantification of TDP-43 in human cerebrospinal fluid, which was impossible by the conventional measurement method of the comparative example. Moreover, it was suggested that, by using a concentration (measured value) of TDP-43 in a human body fluid which concentration was obtained by the measurement method of the example, information which was capable of being used for a biochemical diagnosis of TDP-43 proteinopathy such as ALS or FTLD was obtained, which was conventionally impossible.

### [Evaluation Example 3] Study of ratio of non-capture beads to capture beads

The measurement method of the example enabled highly sensitive quantification of TDP-43 in a human biological sample. One of reasons for this is considered to be a mixing ratio between (i) non-capture beads (helper beads) to each of which an antibody specific to TDP-43 does not bind and each of which does not capture an immune complex and (ii) capture beads to each of which the antibody specific to TDP-43 binds (ratio of the number of the non-capture beads to the capture beads). Therefore, an optimum mixing ratio between the non-capture beads and the capture beads was studied.

TDP-43 was measured by a procedure similar to that in Evaluation Example 1. A human TDP-43 standard peptide was used as a target protein. The human TDP-43 standard peptide was diluted with Sample diluent of Simoa (trademark) Homebrew Assay Development kit so as to be 1.67 pg/mL or 5.0 pg/mL. The diluted human TDP-43 standard peptide was used as a measurement sample. The total number of the capture beads and the non-capture beads was set to approximately 400,000 to 500,000 with respect to 90 pL of the measurement sample. Then, the total number of the capture beads and the non-capture beads was regarded as 100%. A percentage of the number of the non-capture beads was varied to 0%, 50%, and 75%. AEBs in a case where TDP-43 was measured with these percentages were compared. In one measurement, 152 pL of a human TDP-43 standard peptide solution was used.

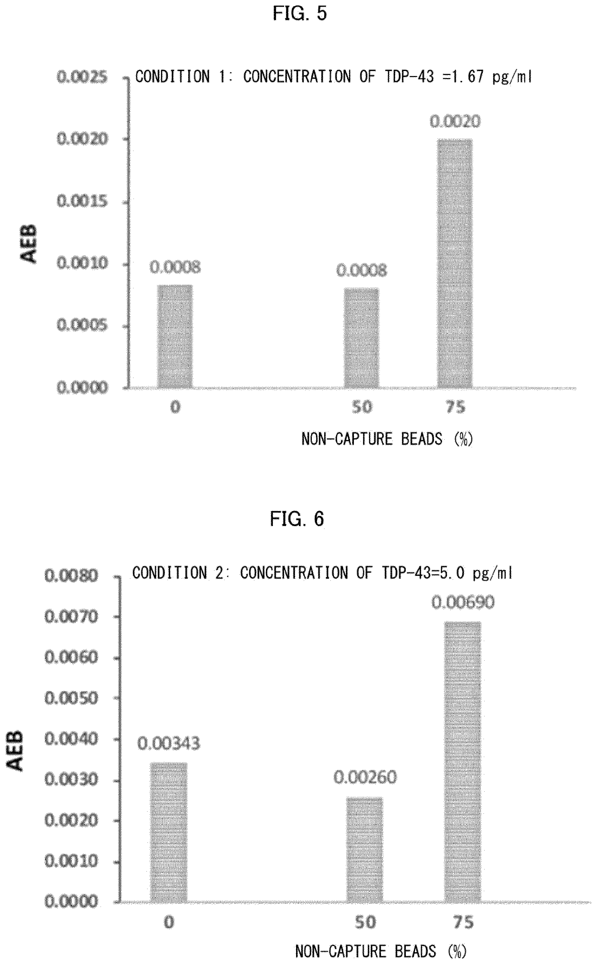

Fig. 5 shows a studying result obtained in a case where a concentration of TDP-43 was 1.67 pg/mL. Fig. 6 shows a studying result obtained in a case where the concentration of TDP-43 was 5.0 pg/mL.

As shown in Figs. 5 and 6, the most intense AEB was obtained in a case where the percentage of the number of the non-capture beads was 75%. It was suggested, from the results in Evaluation Example 3 and previous experience, that, in order to detect TDP-43, the ratio of the capture beads to the non-capture beads (the capture beads : the non-capture beads) preferably fell within a range of 25:75 to 10:90 (i.e., 1:3 to 1:9).

### [Evaluation Example 4] Quantification of phosphorylated TDP-43 in human cerebrospinal fluid (CSF)

Phosphorylated TDP-43 was quantified by the measurement method of the example, with use of human CSF as samples. As a detection antibody, a 5-22-2 antibody was used. The 5-22-2 antibody is a monoclonal antibody that specifically recognizes phosphorylated TDP-43, and was provided by Dr. HASEGAWA Masato of Tokyo Metropolitan Institute of Medical Science. An antigen peptide of the 5-22-2 antibody is a peptide of phosphorylated TDP-43 which peptide corresponds to the 404th to 410th amino acids in the amino acid sequence of TDP-43 (amino acid sequence registered as NCBI Reference Sequence NP_031401) and in which peptide the 409th and 410th serines (S) are phosphorylated. In the measurement method of the example, a concentration of entire beads and a ratio of the number of non-capture beads to the number of capture beads were set similarly to those in Evaluation Example 1.

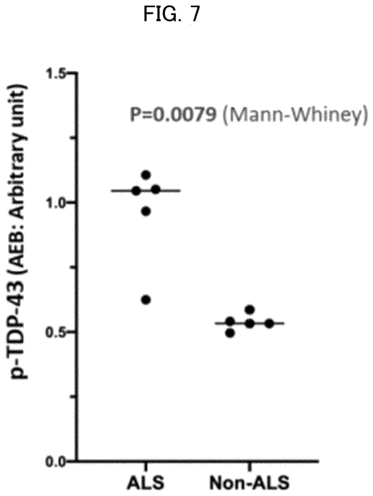

Phosphorylated TDP-43 in the CSF of five patients with amyotrophic lateral sclerosis (ALS), in which abnormally aggregated TDP-43 accumulates within a central nervous system, and four control patients (non-CTRL) was measured. The CSF was diluted four-fold with Sample diluent of Simoa (trademark) Homebrew Assay Development kit, and then measurement was carried out. A buffer was used as a blank. Fig. 7 shows results of quantifying phosphorylated TDP-43. A vertical axis in Fig. 7 indicates signal values (arbitrary unit) of phosphorylated TDP-43 detected in the CSF.

As shown in Fig. 7, by the measurement method of the example, it was possible to detect phosphorylated TDP-43 in the human CSF of all of the samples of the 10 patients. A clear difference was also found between the ALS patients and the control patients (non-ALS) in terms of the concentrations of phosphorylated TDP-43 in the CSF.

It was found from Evaluation Example 4 that the measurement method of the example enabled, in addition of quantification of non-phosphorylated TDP-43 in human cerebrospinal fluid, quantification of phosphorylated TDP-43 in human cerebrospinal fluid, which was impossible by the conventional measurement method of the comparative example. It was suggested that, by the measurement method of the example which enabled quantification of non-phosphorylated TDP-43 and phosphorylated TDP-43, information which was capable of being used for a biochemical diagnosis of TDP-43 proteinopathy such as ALS or FTLD was obtained, which was conventionally impossible.

### [Evaluation Example 5] Suppression of measurement error by addition of EDTA

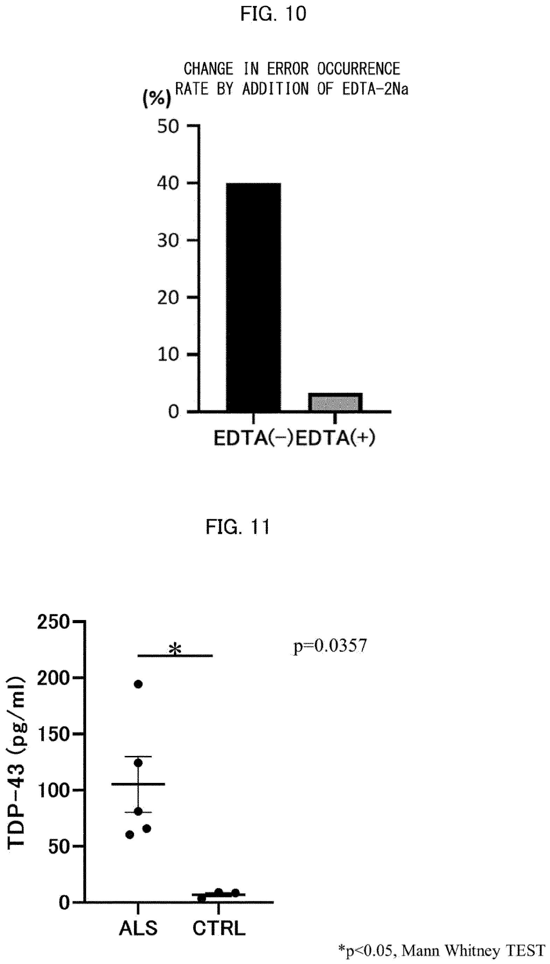

Next, verified was whether or not a measurement error would be suppressed by adding EDTA to a bead buffer used for capture beads. A test was carried out under a condition similar to that in the measurement method of the example, except that a bead buffer used for capture beads was prepared so as to contain 0.1% by mass to 1.0% by mass of EDTA-2Na (disodium dihydrogen ethylenediamine tetraacetate dihydrate). A bead buffer which did not contain EDTA-2Na was used as a buffer with which a comparison was made. A concentration of entire beads and a ratio of the number of non-capture beads to the number of the capture beads were set similarly to those in Evaluation Example 1. As samples, healthy human blood plasma (30 specimens) was used. The blood plasma was diluted four-fold with Sample diluent of Simoa (trademark) Homebrew Assay Development kit, and then measurement was carried out. The measurement was carried out twice per sample. Fig. 10 shows evaluation results.

A graph on the left of Fig. 10 shows a result of not adding EDTA-2Na. A graph on the right of Fig. 10 shows a result of adding EDTA-2Na. An error occurrence rate (%) was calculated by (the number of measurements in which a measurement error occurred) / (the total number of measurements) × 100. As the error occurrence rate becomes lower, a measurement error becomes lower.

As shown in Fig. 10, by adding EDTA-2Na to the bead buffer used for the capture beads, it was possible to significantly reduce the error occurrence rate.

### [Evaluation Example 6] Measurement of concentration of TDP-43 in human blood

TDP-43 was quantified by the measurement method of the example, with use of human blood as samples. As a detection antibody, a 5-22-2 antibody was used. In the measurement method of the example, a concentration of entire beads and a ratio of the number of non-capture beads to the number of capture beads were set similarly to those in Evaluation Example 1.

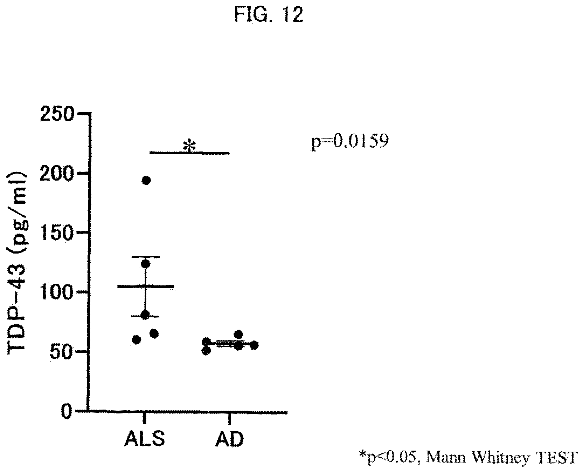

TDP-43 in the blood of five patients with amyotrophic lateral sclerosis (ALS), in which abnormally aggregated TDP-43 accumulates within a central nervous system, and five control patients (CTRL) was measured. TDP-43 in the blood of five patients with Alzheimer's disease (AD) was also measured. By a typical method, blood plasma was separated from the blood collected from the patients. Thereafter, an insoluble matter was removed by centrifugation (10,000 to 15,000×g, 10 minutes). The blood plasma was diluted four-fold with Sample diluent of Simoa (trademark) Homebrew Assay Development kit, and then measurement was carried out. A buffer was used as a blank. Figs. 11 and 12 each show results of quantifying TDP-43.

A vertical axis in each of Figs. 11 and 12 indicates amounts of TDP-43 detected in the blood. The amounts of TDP-43 were calculated by carrying out calibration with a human TDP-43 standard peptide.

Fig. 11 shows results of measuring TDP-43 in the ALS patients and the target patients (CTRL). By the measurement method of the example, it was possible to detect TDP-43 in the human blood of all of the samples of the 10 patients. A clear difference was also found between the ALS patients and the control patients (CTRL) in terms of concentrations of TDP-43 in the blood.

Fig. 12 shows results of measuring TDP-43 in the ALS patients and the AD patients. In the patients with AD, with regard to which it has been reported that cases pathologically involving some degree of TDP-43 deposition exist, the concentrations of TDP-43 in the blood were higher than those in the control patients (CTRL in Fig. 11), but were significantly lower than those in the patients with ALS, which is pure TDP-43 proteinopathy. Therefore, it was found from Fig. 12 that the measurement method of the example was useful for diagnosing TDP-43 proteinopathy such as ALS with use of blood. In addition, there is a possibility that it is also possible to detect TDP-43 deposition which exists as complication pathology of AD and which is in a sub-clinical condition (a condition in which there is no clinical symptom of ALS but deposition pathologically exists).

### Industrial Applicability

The measurement method in accordance with an embodiment of the present invention makes it possible to provide information which can be used for a highly sensitive biochemical diagnosis of TDP-43 proteinopathy such as ALS or FTLD, and can be used, for example, in a pharmaceutical application or the like.

### Reference Signs List

20 Measurement device
21, 51 Processing section (CPU)
22, 52 Main storage section
23, 53 ROM
24, 54 Auxiliary storage section
25, 55 Input I/F
26, 56 Output I/F
27, 57 Media I/F
28, 58 Communication I/F
29, 59 Bus
30, 60 Input section
31, 61 Output section
32, 62 Storage medium
50 Information input device

## Claims

1. A method for measuring TDP-43 in a biological sample, the method comprising:
a preparing step of preparing a measurement sample including (i) non-capture beads and (ii) capture beads to each of which an immune complex of TDP-43 in a biological sample, a capture antibody, and a detection antibody binds, by forming the immune complex on each of the capture beads in the presence of the non-capture beads; and
a detecting step of detecting a signal derived from the immune complex in the measurement sample, wherein
each of the non-capture beads does not bind to the immune complex,
at least one of the detection antibody and the capture antibody specifically recognizes the TDP-43,
an epitope of the capture antibody and an epitope of the detection antibody differ from each other,
a ratio of the number of the non-capture beads to the number of the capture beads is such that the non-capture beads are not less than 2.3 and not more than 9 in a case where the capture beads are regarded as 1, and
a concentration of the capture beads and the non-capture beads is not less than 400,000 beads and not more than 500,000 beads with respect to 90 pL of the measurement sample.

2. A method for measuring TDP-43 in a biological sample, the method comprising:
a preparing step of preparing a measurement sample including (i) non-capture beads and (ii) capture beads to each of which an immune complex of TDP-43 in a biological sample, a capture antibody, and a detection antibody binds, by forming the immune complex on each of the capture beads in the presence of the non-capture beads; and
a detecting step of detecting a signal derived from the immune complex in the measurement sample, wherein
each of the non-capture beads does not bind to the immune complex,
at least one of the detection antibody and the capture antibody specifically recognizes the TDP-43,
an epitope of the capture antibody and an epitope of the detection antibody differ from each other,
a ratio of the number of the non-capture beads to the number of the capture beads is such that the non-capture beads are not less than 2.3 and not more than 9 in a case where the capture beads are regarded as 1,
a concentration of the capture beads and the non-capture beads is not less than 400,000 beads and not more than 500,000 beads with respect to 90 pL of the measurement sample, and
EDTA is added to a buffer solution used for the capture beads and the non-capture beads.

3. The method as set forth in claim 1 or 2, wherein the TDP-43 is phosphorylated TDP-43.

4. The method as set forth in any one of claims 1 through 3, wherein a concentration of the TDP-43 contained in the measurement sample is not less than 1.43 pg/mL and not more than 6.65 pg/mL.

5. The method as set forth in any one of claims 1 through 4, wherein
one of the epitope of the detection antibody and the epitope of the capture antibody includes a peptide composed of the 203rd to 209th amino acids in an amino acid sequence represented by SEQ ID NO. 1, and
the other of the epitope of the detection antibody and the epitope of the capture antibody is (i) an epitope included in a peptide composed of the 261st to 414th amino acids in the amino acid sequence represented by SEQ ID NO. 1 or (ii) an epitope including a peptide which is composed of the 404th to 410th amino acids in the amino acid sequence represented by SEQ ID NO. 1 and in which the 409th and 410th serines are phosphorylated.

6. The method as set forth in any one of claims 1 through 5, wherein
in the detecting step,
the measurement sample is brought into contact with a substrate having a plurality of microwells,
the capture beads and the non-capture beads in the measurement sample are respectively placed in the plurality of microwells,
the signal derived from the immune complex on each of the capture beads placed in corresponding ones of the plurality of microwells is detected, and
each of the plurality of microwells has a volume which allows merely one of the capture beads or the non-capture beads to be held in the each of the plurality of microwells.

7. The method as set forth in any one of claims 1 through 6, wherein, in the detecting step, detection of the signal is carried out by capturing an image of the signal derived from the immune complex.

8. The method as set forth in any one of claims 1 through 7, wherein the biological sample is spinal fluid or a blood sample.

9. The method as set forth in claim 8, wherein the blood sample is blood serum or blood plasma.

10. The method as set forth in any one of claims 1 through 9, wherein
the immune complex is a complex of the capture antibody, the TDP-43 in the biological sample, and the detection antibody, and
the capture antibody binds to each of the capture beads.

11. The method as set forth in any one of claims 1 through 10, wherein the immune complex is formed by mixing the capture antibody which binds to each of the capture beads, the biological sample which contains the TDP-43, and the detection antibody in the presence of the non-capture beads.

12. A method for obtaining information on whether or not a subject is affected by TDP-43 proteinopathy, on the basis of an intensity of a signal obtained by the method recited in any one of claims 1 through 11.

13. A reagent or reagent kit used in the method recited in any one of claims 1 through 12, the reagent or reagent kit comprising the capture beads and the non-capture beads.

14. A device which measures TDP-43 in a biological sample collected from a subject, the device comprising
a processing section, wherein
the processing section obtains a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads,
each of the non-capture beads does not bind to the immune complex,
at least one of the detection antibody and the capture antibody specifically recognizes the TDP-43,
an epitope of the capture antibody and an epitope of the detection antibody differ from each other,
a ratio of the number of the non-capture beads to the number of the capture beads is such that the non-capture beads are not less than 2.3 and not more than 9 in a case where the capture beads are regarded as 1, and
a concentration of the capture beads and the non-capture beads is not less than 400,000 beads and not more than 500,000 beads with respect to 90 pL of the measurement sample.

15. A device which obtains information on whether or not a subject has TDP-43 proteinopathy, on the basis of an intensity of a signal derived from TDP-43 in a biological sample collected from the subject, the device comprising
a processing section, wherein
the processing section obtains a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads,
each of the non-capture beads does not bind to the immune complex,
at least one of the detection antibody and the capture antibody specifically recognizes the TDP-43,
an epitope of the capture antibody and an epitope of the detection antibody differ from each other,
the processing device obtains information on whether or not a subject has TDP-43 proteinopathy, on the basis of an intensity of the signal,
a ratio of the number of the non-capture beads to the number of the capture beads is such that the non-capture beads are not less than 2.3 and not more than 9 in a case where the capture beads are regarded as 1, and
a concentration of the capture beads and the non-capture beads is not less than 400,000 beads and not more than 500,000 beads with respect to 90 pL of the measurement sample.

16. A computer program for measuring TDP-43, the computer program causing a computer to carry out
a step of obtaining a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads, wherein
the biological sample is collected from a subject,
each of the non-capture beads does not bind to the immune complex,
at least one of the detection antibody and the capture antibody specifically recognizes the TDP-43,
an epitope of the capture antibody and an epitope of the detection antibody differ from each other,
a ratio of the number of the non-capture beads to the number of the capture beads is such that the non-capture beads are not less than 2.3 and not more than 9 in a case where the capture beads are regarded as 1, and
a concentration of the capture beads and the non-capture beads is not less than 400,000 beads and not more than 500,000 beads with respect to 90 pL of the measurement sample.

17. A computer program for obtaining information on whether or not a subject has TDP-43 proteinopathy, the computer program causing a computer to carry out:
a step of obtaining a signal derived from an immune complex of TDP-43 in a biological sample, a detection antibody, and a capture antibody, with regard to a measurement sample that includes (i) non-capture beads and (ii) capture beads to each of which the immune complex binds and that has been prepared by forming the immune complex on each of the capture beads in the presence of the non-capture beads; and
a step of obtaining information on whether or not a subject from whom the biological sample has been collected has TDP-43 proteinopathy, on the basis of an intensity of the signal, wherein
each of the non-capture beads does not bind to the immune complex,
at least one of the detection antibody and the capture antibody specifically recognizes the TDP-43,
an epitope of the capture antibody and an epitope of the detection antibody differ from each other,
a ratio of the number of the non-capture beads to the number of the capture beads is such that the non-capture beads are not less than 2.3 and not more than 9 in a case where the capture beads are regarded as 1, and
a concentration of the capture beads and the non-capture beads is not less than 400,000 beads and not more than 500,000 beads with respect to 90 pL of the measurement sample.
